# EUROPEAN PATENT APPLICATION

(11) **EP 2 065 820 A1**
(43) Date of publication of application: **03.06.2009**
(21) Application number: 07121217.9
(22) Date of filing: 21.11.2007
(51) Int. Cl.: G06F 19/00

(54) **Foreign study catalog**

(71) Applicant: Agfa HealthCare NV, 2640 Mortsel (BE)
(72) Inventor: Van Dokkumburg ,Steven, St. Clemens Ontario N0B 2M0 (CA); Ho, Kinson, Waterloo Ontario N2K 3G9 (CA); Mayo, Peter, Baden Ontario N3A 3K7 (CA); Eppel, Michael, Kitchener Ontario N2M 1J4 (CA)

(57) **Abstract**

A system and method for creating a central information reference point for a host of applications within the hospital environment, for giving an accurate representation of all sources of information within the deployment by ensuring the collected information is up to date and normalized, and for producing the information in a complete, fast, predictable and consistent way.

## Description

### FIELD OF THE INVENTION

The present invention relates to data managing in medical systems. More specifically the invention is related to the centralization, the accurateness, the normalization, and the reproduction of information.

### BACKGROUND OF THE INVENTION

Within the healthcare business space there is a trend that sees hospitals joining forces to create larger conglomerates of healthcare services. Some of these mergers can be attributed to budgetary and funding changes, others to having certain geographical institutions offering more specialty-oriented services. Still others are an attempt to load balance patient traffic within a geographic region.

Regardless of the reason, the term "Enterprise" has been introduced to refer to this conglomerate of hospitals so that a unified solution can be achieved. However, "Enterprise" does not inherently mean that:
- the entire enterprise runs on a single data store or database
- all the institutions cooperating in this conglomerate run software from the same vendor
- every institution uses the same nomenclature for demographic descriptions

So there is the need for a seamless integration of these disparate systems so that healthcare professionals can operate as though they are working within a single system. This requires the collection, distribution and presentation of data to the users as though there is a single RIS (Radiology Information System), a single PACS (Picture Archiving and Communication System), a single reporting system, and so on.

Historically, a single site has been constructed of a number of departmental information systems (RIS, PACS, CIS, ...) and a number of modalities. When users of a PACS system were interested in a patient's imaging history, they needed only to consult their own PACS system. When the formation of a conglomerate healthcare service (an enterprise) is created, a user of the PACS system must consider other information that exists in places other than his/her own local PACS system.

In order to create this detailed enterprise-wide view of a patient record, multiple points of reference must be consulted. Each system must be aware of every other system participating in the enterprise. This means that the user must perform individual queries against each system to get a complete picture.

By introducing a single point of reference, the user would only need to consult one place. The solution to this problem would have to ensure that all sources of information have been consulted in order to present a unified view to the user. Additionally, the response to the user must be faster than if the user performed the individual queries him/herself.

Although there may be many different vendors participating in the healthcare enterprise, there must be agreement on how information can be passed from one branch of the enterprise to another. Although other protocols can be added to the list, primary conversations between PACS systems are DICOM (Digital Imaging and Communications in Medicine).

There are, however, drawbacks to using a standard protocol. Many PACS systems use custom extensions to make sorting and grouping of information easier for their own workstations. Much of this value-added data is lost when it is forced through a narrowed standard mechanism.

For example, a PACS vendor may keep track of dosages of contrast agent administered during an examination and use that information to link to a laboratory information system. However, when this same PACS system is queried for information about the study, they do not provide the dosage information in their response. This shows that using the standard mechanisms for querying for information may be reduced to the lowest common denominator.

Typical queries for PACS users include searching for previous examinations for a patient that are similar or related to the examination in question. For example, if a user is looking at a recent examination of a woman's second trimester ultrasound, the examinations pertaining to her previous pregnancy or a motor vehicle accident where her pelvis was broken are considered relevant while examinations showing a broken arm from a tumble out of a tree at the age of three would not be considered relevant.

This is a fairly simple problem to solve if all studies use the same nomenclature for study details. It would be simple to construct a query that said, "Show me all the studies for this patient that are related pelvis". But what happens if one system refers to that area of the body as "pelvis" and another calls it "pelvic region"? What about if one system uses French ("bassin") as the primary language, while another uses English ("pelvis")?

Experience in the Healthcare world indicates that nearly every site uses their own custom names for body parts, specialties and procedure descriptions. This means that when these sites are combined together to create an enterprise, some kind of normalization needs to occur in order to ensure that relevant priors can be obtained.

### SUMMARY OF THE INVENTION

The above-mentioned problems are solved by the Foreign Study Catalog (FSC) having the specific features set out in claim 1. Specific features for preferred embodiments of the invention are set out in the dependent claims.

### BRIEF DESCRIPTION OF THE DRAWINGS

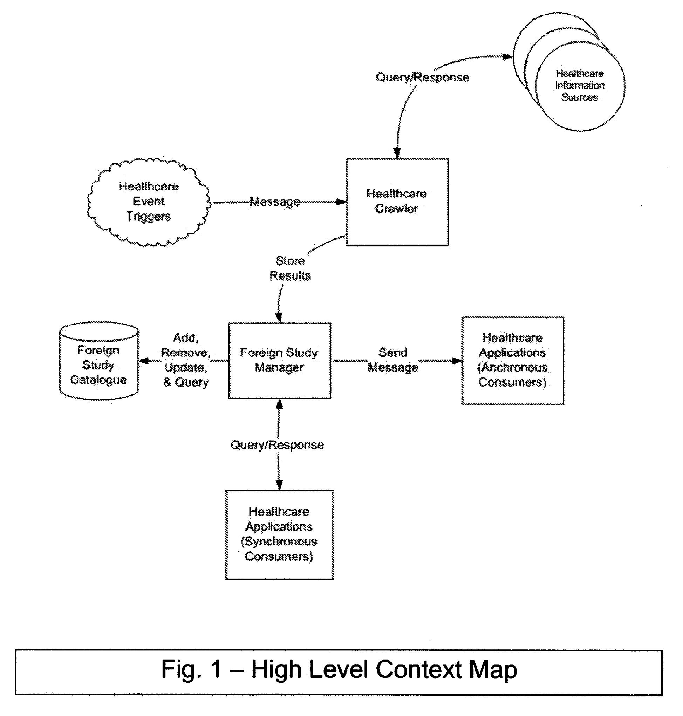

Fig. 1 shows the high level context map.

### DETAILED DESCRIPTION OF THE INVENTION

Healthcare information sources are typically other PACS systems. Communication with these systems is typically done through DICOM, but other protocols are also possible. Each site that participates in the healthcare enterprise may have one central representative system that the other sites may query. In this way, if there are five sites participating in the healthcare conglomerate, there are five healthcare information sources for the Healthcare Crawler (see below) to consult.

Healthcare event triggers come in the form of DICOM MPPS (Modality Performed Procedure Step) messages or DICOM DM (Detached Management) messages. These messages are fed to the Healthcare Crawler as indicators of what is happening in the healthcare enterprise. The originating source of these messages could be PACS systems, RIS systems, modalities or other DICOM compliant devices.

Although some messages contain procedure and study information, what is common for each of these messages is that they will all contain patient level information.

What follows is a small list of some of the more typical healthcare triggers that the Healthcare Crawler would be interested in and why this solution would be interested in them:
- ***Study Scheduled Event***
   When a study is scheduled within an institution, the Healthcare Crawler searches the Healthcare Information Sources for other information that may be pertinent to the work about to be done. The crawler will constrain its searching by the patient identification parameters so large queries are minimized.
- ***Study Update Event***
   When there have been changes made to study related information, these messages float around the system to ensure that all interested parties are up to date with the changes.
- ***Patient Update***
   Changes to a patient's demographic information would trigger these messages to be sent around the healthcare system. For example, when a patient changes their name, they would update the name in the RIS. The RIS would then send out the update messages to the PACS via some kind of interface engine so that the appropriate records on the PACS get updated as well.
- ***Order Cancelled***
   If a patient had an exam scheduled that was no longer needed, an order cancelled message would be sent out to notify the interested parties.

The Healthcare Crawler is a background server process that formulates queries based on incoming triggers. When the Healthcare Crawler receives a trigger, it parses the patient identification information (i.e. the topic) from the trigger and runs through each of the Healthcare Information Sources and collects information about the topic. It then hands all the raw data to the Foreign Study Manager (see below) where it gets normalized and stored into the Foreign Study Catalogue.

It is important to remember that the Healthcare Crawler performs queries for text-based information. It fetches information about which servers have what image data so that a subsequent service can know where to get the images if needed. The Healthcare Crawler will ignore the study and procedural level information since it fetches all the information it can about the patient. A subsequent service will determine which of the study information retrieved is relevant for image retrieval. Information within scope of the Healthcare Crawler's include demographic data, report data, outstanding orders, or any other information that may be relevant for a subsequent service downstream to know.

The main reason for the Healthcare Crawler to exist is to perform the queries before the user of the workstation even knows what to ask. The Healthcare Crawler will be aware of a new order minutes, hours or even days before the physician responsible for creating the report for the images. The Healthcare Crawler is able to search throughout the system and collect all needed information. When the physician is ready to examine the images, he needs only to ask the FSC for the information rather than query all external systems.

Not only does the Healthcare Crawler save the end user time for query responses, but it also improves the overall performance of the enterprise. There are three reasons for this:
1. ***Queries are less expensive than retrieves.***
   By querying for all information about the topic and normalizing the data into a single nomenclature, better decisions can be made on what studies need to be retrieved. The alternate case is to retrieve all studies just in case it is something that the physician needs. Or, the converse, retrieve only minimal amounts of information and run the risk of not providing the physician with the "full picture".
2. ***Fewer queries are being performed over long distances.***
   Although the Healthcare Crawler is performing queries for each trigger it receives, there are less queries being performed over all. End users may be sharing worklists that look across the enterprise for information. In many cases, these worklists are asking for the exact same information, but since there is no central sub-system performing the queries on behalf of the users, each user is performing their own queries across the enterprise. If the worklists are pointed at the FSC, every user will only be making local queries rather than over the enterprise topology.
3. ***Queries are well constrained.***
   As mentioned earlier in this section, queries are primarily constrained on the topic's identifiers. This means that each query will only produce results for a single patient. Typical worklists don't know specific patient details so they need to query using much broader criteria. For example, an enterprise worklist may ask for all new CTs for today. Performing this query against the enterprise is a large undertaking and will cause many subsystems to generate large queries. However, if this same query is focused only on the FSC (a single system that is representative of the entire enterprise) will prove more performant.

The Foreign Study Manager performs three main rolls:
1. Expose a public interface to the content of the FSC solution
2. Provide an entry point for the Healthcare Crawler to store information into the FSC Data Store
3. Normalize incoming information using translation rules
4. Ensure that the data provided is as up to date as possible

All of data coming from the Healthcare Crawler is normalized at the Foreign Study Manager. The Foreign Study Manager is the component that is configured with a set of rules for how to translate data. The rules that govern this translation are rooted in where the data is coming from. For example, study descriptions that are returned from one Healthcare Information Source could be different in structure and nomenclature than those returned from another Healthcare Information Source. One source could refer to a scan of the brain as a "brain scan" while another source could refer to it as a "head scan". The FSC will normalize this discrepancy based on an internal set of rules. Thus regardless of whether a calling application asks for a "head" or a "brain" they will get the same results back.

Healthcare Applications are any software packages that make use of the functionality of the FSC solution. These users are further classified as Synchronous and Asynchronous Consumers.

Synchronous Consumers are those software packages that ask for information of the FSC that requires an immediate answer. For example, a client workstation asking for information is processed with the highest priority by the Foreign Study Manager since a user is waiting for a response. The Foreign Study Manager will handle the query for information by examining the FSC Data Store and determine the age of the information. If the data is new enough, it will hand the information back to the querying Healthcare Application. If the resultant data is ***not new*** enough, it will hand a task to the Healthcare Crawler to fetch the information from the Healthcare Information Sources. The Healthcare Crawler will then pass the results back to the Foreign Study Manager which will normalize and update the FSC Data Store. The FSC Data Store is once again queried and the information is passed back to the querying Healthcare Application. The FSC thus ensures that the data it is holding is as up to date as possible. A set of rules that define what "stale" means are used to set a threshold for what it means to be current. This determination is done at several points in the data flow, but will be mainly triggered when using applications query the FSC.

Asynchronous Consumers are Healthcare Applications that do not query, but merely accept triggers from the Foreign Study Manager. An example of such a Healthcare Application would be an imaging prefetch engine: As triggers come into the Healthcare Crawler, queries will be dispatched to all Healthcare Information Sources. As the results come back, they will be passed to the Foreign Study Manager for normalization and storage. Messages will then be sent to the Asynchronous Healthcare Applications so they get notified of the Healthcare Event that just occurred.

The Foreign Study Catalogue Data Store is an off-the-shelf database management system like Microsoft SQL Server or Oracle. The interface to the database engine is standard Open Database Connectors (ODBC).

Having described in detail preferred embodiments of the current invention, it will now be apparent to those skilled in the art that numerous modifications can be made therein without departing from the scope of the invention as defined in the appending claims.

## Claims

1. An integration method for disparate systems in the healthcare environment, said method **characterized by**:
(a) creating a central information reference point;
(b) accepting data for centralization;
(c) storing said data; and
(d) producing said data on request.

2. The method of claim 1, wherein, after (a), data is pre-fetched by a crawler process comprising:
i) receiving an event trigger;
ii) parsing the topic from the event trigger; and
iii) collecting information about the topic.

3. The method of claims 1 to 2, wherein (d) is comprising:
i) receiving a request for information;
ii) examining the data store for said information;
iii) if (ii) found, determining the age of said information;
iv) if (ii) found, and said age is below a threshold, passing said information to the requesting application;
v) if (ii) not found, or said age is above said threshold:
(a) passing a task to the crawler process to fetch said information;
(b) updating the data store with the information returned by said crawler process; and
(c) passing said information to the requesting application.

4. The method of claims 1 to 3, wherein, before storing or updating said data, said data is normalized by a normalization process configured with a set of normalization rules.

5. A computer-readable medium upon which a plurality of instructions are stored, the instructions for performing the steps of the method as claimed in claim 1.

6. A system for the integration of disparate systems in the healthcare environment, said system **characterized by**:
(a) a memory for storing a plurality of instructions; and
(b) a processor coupled to the memory, said processor configured for:
i) creating a central information reference point;
ii) accepting data for centralization;
iii) storing said data; and
iv) producing said data on request.

7. The system of claim 6, wherein, after (i), data is pre-fetched by a crawler process comprising:
(a) receiving an event trigger;
(b) parsing the topic from the event trigger; and
(c) collecting information about the topic.

8. The system of claims 6 to 7, wherein (iv) is comprising:
(a) receiving a request for information;
(b) examining the data store for said information;
(c) if found, determining the age of said information;
(d) if found, and said age is below a threshold, passing said information to the requesting application;
(e) if not found, or said age is above said threshold:
(i) passing a task to the crawler process to fetch said information;
(ii) updating the data store with the information returned by said crawler process; and
(iii) passing said information to the requesting application.

9. The method of claims 6 to 8, wherein, before storing or updating said data, said data is normalized by a normalization process configured with a set of normalization rules.
